# EUROPEAN PATENT APPLICATION

(11) **EP 1 889 921 A2**
(43) Date of publication of application: **20.02.2008**
(21) Application number: 05801662.7
(22) Date of filing: 04.10.2005
(51) Int. Cl.: C12Q 1/68

(54) **SOLUTION FOR THE INDEFINITE MAINTENANCE OF NUCLEIC ACIDS IN THE CELL OF ORIGIN THEREOF**

(30) Priority: 05.10.2004 ES 200402365
(71) Applicant: Universidad Complutense de Madrid Rectorado, 28040 Madrid (ES)
(72) Inventor: DUNNER BOXBERGER, Susana Universidad Complutense, 28040 Madrid (ES); CAÑON FERRERAS, Javier UniversidadComplutense, 28040 Madrid (ES)
(74) Representative: Carpintero Lopez, Francisco
(86) International application number: PCT/ES2005/000531
(87) International publication number: WO 2006/040376

(57) **Abstract**

The invention relates to a product having characteristics which enable a biological sample obtained from an individual to be maintained under conditions such that the quality of nucleic acids later extracted is not affected, thereby guaranteeing the integrity of the sample regardless of the time that elapses between the sample being obtained and the nucleic acid being extracted. For said purpose, a solid (organic) or liquid (blood, semen, cell culture etc.) biological sample from a eukaryote or prokaryote is mixed in a solution comprising a mixture of trisodium ethylenediaminetetraacetic acid (EDTA), sodium fluoride (NaF), and saturation with thymol (extracted from *Thymus vulgaris* L.) in order to prevent contamination and bacterial growth and adjustment to a pH of 8. Said solution can be used to collect samples and to maintain said samples therein at ambient temperature for an indefinite period, for the later extraction of nucleic acids and PCR, cloning, sequencing, hybridisation or mutagenesis with sufficient integrity and reliability guarantees.

## Description

### Background of the invention

The present disclosure relates to the field of molecular biology, more particularly to methods, reagents and systems for preserving nucleic acids (DNA, RNA, cDNA, mitDNA) in their cell of origin belonging to solid tissues (organs) and also to liquids (body fluids) for an indefinite period and at ambient temperature, maintaining an optimum quality.

### State of the technique

The diagnostic analysis or tests based on nucleic acids (DNA or RNA) are getting more frequent in clinical laboratories, hospitals, veterinarian clinics etc, and allow the genetic identification of any individual, to establish their genetic relationships with their sibs (mother, father, son) or with individuals belonging to other populations (within breeds, species, genus, family or class), to detect if it is bearing a mutation at a particular gene, to analyse the presence of an pathogen, predict the type of tumour etc.

Most of these tests are done indirectly analysing particular genetic markers, but in the last years the use of Polymerase Chain Reaction or PCR (Mullis et al., Patents n° US4,683,195, US4,683,202, US4,800,159 and US4,965,188) has allowed to do more specific, sensible and cost effective analysis from a molecular perspective.

To be able to perform these analysis, a good quality nucleic acid is necessary, that is a nucleic acid of double strand, not degraded nor fragmented.

Many are the factors affecting the quality of DNA samples (type of the tissue maintained, type of additive used, length and form of transport to the laboratory) and the stability of the analysed markers, so it is important to decide which will be the conservation method before starting with a DNA bank.

There are many references in the literature dealing with DNA conservation with no additive and at ambient temperature: in amniotic liquid samples for 8 days (Casareale et al., 1992. PCR Methods Appl. Nov;2(2):149-53), in blood results vary from 24h (Polakova et al., 1989, Bratisl Lek Listy. Nov;90(11):844-7), to a maximum of four weeks (Joss and Do, 1997. J.Med Microbiol, Jan;46(1):92-6), in blood deposited and dried on a glass slide (Aggarwal, 1992. Pharmacotherapy. Aug;22(8):954-60), on bucal wash maintained during more then 60-90 days at room temperature (Andrisin, 2002. Pharmacotherapy.Aug;22(8):954-60) all the references giving negative results. The experiments done at higher temperatures (23°C during one week (Madisen et al., 1987. Am J Med Genet.Jun;27(2):379-90) or at 37°C (Cushwa and Medrano, 1993. Biotechniques, 14(2):204-7) also produced a degraded nucleic acid. When an additive is used, DNA keeps good conditions after 72 weeks when the sample is mixed to phenol (Albarino and Romanowski, 1994. Mol Cell Probes. Oct;8(5):423-7), with another additive called LST (Low cost Storage and Transportation buffer) which maintains the samples for a maximum of four weeks at room temperature before freezing if more time is needed (Schultz, 1999. Am J Clin Pathol. Jun;111(6):748.52). Fukatsu (1999. Mol Ecol. Nov;8(11):1935-45) observes a good DNA quality after six months conservation of the samples in acetone, ethanol, 2-propanol, diethyl ether acetate.

There is also another form of conserving DNA in their cells by maintaining blood on a piece of paper. There is a commercial availability of different types of cards for this purpose: FTA cards from which many references can be found with very different results. So Natarajan et al., 2000. (Biotechniques. Dec;29(6):1328-33) not specifying time of conservation, produce satisfactory quality results but other authors show a lower sensibility over time to detect the presence of pathogens DNA such as malaria (Zhong and al., 2001. J Clin Microbiol. Mar;39(3):1195-6); same occurs with the Isocode STIX cards also commercially available. A third work reflects the fact that blood on FTA cards maintains the sample to extract DNA after one year whenever conservation temperature is -20°C and two to three months at room temperature (Natarajan et al., 2000. Biotechniques. Dec;29(6):1328-33). Guthrie cards are paper cards in which blood from new borns is deposited with the aim of analysing metabolic alterations and following Makowski (2003. Ann Clin Lab Sci. Summer;33(3):243-50) can be a good source of DNA as they allow to conserve blood during many years. Sierra Diagnostics uses a method based on an amount of a divalent metal chelator selected from EDTA, EGTA or BAPTA at low molarities (in the range of from about 0.001 M to 0.1M) mixed with lithium chloride, guanidine or sodium salicylate or sodium perchlorate, and sodium thiocyanate to conserve urine samples for many hours. With this patented method (US006458546B1) they maintain urine samples from their collection until DNA extraction for a period of maximum ten days.

Although description of all these methods, the most used to maintain samples during long periods with no loose of DNA quality is to freeze it at different temperatures: at -18°C during 7 days and 3 months, six months at 70°C after thawing many times (Naber, 1996, Diagn Mol Pathol. Dec;5(4):253-9), or at least two months at -70°C (Madisen et al., 1987. Am J Med Genet. Jun;27(2):379-90) and the use of this last temperature is recommended to maintain tissues and blood and only DNA is extracted when there is a need (Holland et al., 2002. Mutation Res. 7702:1-18).

Collection of blood samples in field conditions can only be performed with an anticoagulant (generally EDTAK₃ - Nielsen, 1985. Acta Pathol Microbiol Immunol Scand [C]. Apr;93(2):49-52) at room temperature and shortening time to get to the laboratory and extracting the DNA. This time can be much larger if this sample is refrigerated to a temperature of 5°C. In these conditions, DNA extraction must be immediate upon arrival to the laboratory to avoid degradation or the sample must be frozen to allow the integrity of the nucleic acid contained to be maintained. Because of this, in the laboratories there is a tendency to extract the nucleic acid of interest of the sample at reception to avoid degradation and ensure analysis accuracy. These needs of immediate extraction implicate an uncertainty in the tasks assignation in the laboratory as these are conditioned to the entry of an unknown number of samples.

The addition of antiseptic reagent in the collection solution, would lengthen the half life of the nucleic acids in their cell at ambient temperature, avoiding bacterial growth and activity of nucleases responsible of their degradation. However, there is no reference giving this possibility. Thymol is an antiseptic and disinfectant which destroys vitality of live ferments, annulates the enzymes present in the medium, prevents from putrefaction and brakes this when initiated. A small quantity of thymol added to albumin, milk, gelatine will keep it for months (Harvey Wickes Felter, MD, and John uri Lloyd, Phr. M., Ph.D.; 1898).

Thymol has been used as a disinfectant dissolved in water (Giraldes, M), as a basis to do inhalations when respiratory affections (Bouillhon and Paquet), to treat diphtheria and other illness produced by microorganisms, articular rheuma, typhus, phthisis and pielitis, also as an helminthic and to treat cutaneous problems in the case of eczema, psoriasis or burnings. Nowadays it is used nearly exclusively in collutory solutions for the gingivitis treatment, prepared to inhalations associated to camphor, for topic use liniments, as a topic anaesthetic (http://www.uhe.com/thymol.htm) and in general as an antiseptic and antioxidant component in the medical practice and in the industry as a stabiliser of different therapeutic agents as halothane for anaesthetics (Effect of thymol on kinetic properties of Ca and K currents in rat skeletal muscle. Szentandrássy et al., BMC Pharmacology 2003, 3:9), and finally in the varroasis treatment in the bee, being a product which is not so harmful as a synthetic acaricide as is the oxalic acid (Imdorf, A et al., 1995. Toxicity of thymol, camphor, menthol and eucalyptol in Varroa jacobsoni and Apis mellifera in laboratory tests. Apidologie 26. p27-31).

Very often, sample collection is done in places very far away from the laboratories where these will be processed, in difficult conditions (high temperatures, difficulties to use a refrigerator) which difficults not only the freezing of the sample, but also its immediate refrigeration.

So, it seems necessary to have the availability of an additive which once mixed to any type of tissue (which can be a organ fragment, a vaginal swab etc.) or from any fluid (blood, semen, amniotic liquid etc.) which will maintain the samples in such conditions that the nucleic acids included in the cells will stay in the same conditions for an indefinite period, even at ambient temperature.

### SUMMARY OF THE INVENTION

### Solution for the indefinite maintenance of nucleic acids in the cell of origin thereof

The present disclosure relates to a product which characteristics allow to maintain a sample collected from an individuals, in such conditions that quality of nucleic acids extracted later will not be affected, guaranteeing its integrity independently of the time elapsed between the sample collection and the extraction of the nucleic acid and the temperature at which the sample has been collected. Concretely, it refers to a solution for the indefinite maintenance of nucleic acids in their cell of origin which allows to maintain any biological sample obtained from an individual being this an eukaryote or a prokaryote at ambient temperature or at a temperature equivalent or superior to 5°C.

This is possible by mixing the sample with a solution containing EDTA, NaF and thymol and equilibrating the mix to pH = 8. EDTA is a potent divalent metal chelator which acts eliminating cations which are components of enzymes metal-dependent as are DNAses susceptible of provoking degradation of nucleic acids; its presence will inactivate these enzymes. NaF is the second component present in the solution being another chelator which helps to inactivate enzymes as ligases, polymerases, exonucleases, kinases, nucleases and its presence improves the chelating capability of the EDTA. The third component integrating the solution is Thymol which is an essential oil extracted from the thyme *(Thymus vulgaris* L.) and which has a high antiseptic power, antifungal and antihelmintic. Thymol (or thyme camphor) also called isopropyl-metacresol, 6-isopropyl-m-creol, 3-hydroxy-p-cymene, isopropyl cresol, 2 isopropyl-5-methylphenol, has a molecular formula C₁₀H₁₄₀ and is a crystalline phenol obtained from the volatile oils of *Thymus vulgaris,* Linn (N.O. Labiatae), *Monarda punctata,* Linn. (N.O. Labiatae), *Carum copticum,* Benth. And Hook, f. (N.O.Umbelliferae), and other plants.

Thymol looks like phenol in its action mode although due to its insolubility in body fluids, it is absorbed in a much slower way. It is less irritant and its germicide action is higher than that of phenol. Thymol is a potent bactericide much superior to the rest of phenols ("Factors that interact with the antibacterial action of thyme essential oil and its active constituents". Juven et al., Appl Bacteriol. 1994. Jun;76(6):626-31). Thymol acts on the cellular membrane producing a lack of membrane selective permeability by changing its physical properties. This action on the membranes is common to all phenols and is due to the coexistence of an hydrophilic and a lipophilic region. Thymol produces insoluble proteinates due to the enzymatic inactivation and the protein denaturation, and is the factor which avoids degradation of nucleic acids through the presence of enzymes and thus allows their integrity through long periods of time. Methyl group and isopropil chain improves the antiseptic activity in a more efficient way than phenol as thymol toxicity is lower than that of the phenol.

The product is performed by mixing in a solution an amount of a divalent metals chelator EDTA (Ethylen-diamine-tetraacetic acid) at a molarity varying between 0,39M and 0.56 M in its trisodium form; a component improving chelating capacity of EDTA which is Sodium Fluoride (NaF) which varies between 0.33 M and 0.48 M and the saturation of this solution with Thymol (less than 0.0001 M) to avoid microorganisms growth in the sample while the latter is maintained at temperatures equal or higher then +5°C and its pH equilibration to a value of 8.

The solution acts on the cellular membrane producing changes in the physical properties which make it loose permeability, it also inactivates degrading enzymes by chelating their cations and contributes to the protein denaturation producing insoluble proteinates.

The solution added to any tissue, body fluid, tumour tissue, skin, bone marrow, feather, hair, blood, blood serum, amniotic liquid, egg, semen, saliva, vaginal fluid, sweat, allows the to keep the samples and to maintain nucleic acids included in those during indefinite time at any temperature higher than 5°C. This solution avoids the enzymatic destruction of the nucleic acids and avoids bacterial, fungal or helmintic growth which origin is not due to the sample and which appears together with enzymes which degrade nucleic acids of interest including their proper DNA which presence could biase the results which could be performed on the sample.

The invention reveals that in order to maintaining a nucleic acid in its origin tissue (a fragment of tissue of an organ, of skin, feather, blood, semen, amniotic liquid, vaginal fluid etc.) it is necessary to submerge the sample in a solution containing EDTA (ethylene-diamine-tetraacetic acid) in its trisodium form and at a concentration not lower than 0.39 M, Sodium fluoride (NaF) at a concentration not lower then 0.33 M and saturation of the solution with Thymol and its equilibration to a pH = 8. The nucleic acids referred in this invention are any nucleic acid: deoxyribonucleic acid (DNA), ribonucleic acid (RNA), coding deoxyribonucleic acid (cDNA) and mitochondrial deoxyribonucleic acid (mitDNA) contained in any eukaryote or prokaryote cell.

Mixing the solution with a biologic sample allows to preserve the latter in such conditions that the nucleic acid quality extracted a few hours or years later will not be affected, guaranteeing its integrity independently of the time elapsed between the obtention of the sample and the nucleic acid extraction and guarantying that the processes which will be performed, amplification through PCR, sequencing, cloning, hybridisation, mutagenesis or similar techniques will have a high quality and liability near to 100%. This solution can be prepared in a liquid, solid or micronised form and allows to preserve indefinitely nucleic acids in tissue and body fluids samples in eukaryotes, specially in vertebrates and in prokaryotes.

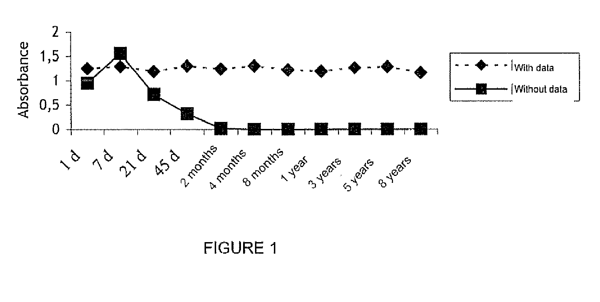

The ability of the described procedure to maintain a nucleic acid in the cell in such conditions that any type of analysis can be successfully performed independently of the time elapsed since sampling has been done, can be observed by comparing samples which are included in the solution object of the invention and samples which are not, both preserved at +5°C, through the use of the absorbance measure given by the nucleic acids after their extraction at different times from sampling as a quality parameter. It can be seen that independently of the time elapsed, samples conserved in the solution disclosed herein reach equivalent quantities of DNA, but samples not maintained in the solution disclosed herein, will show and increase in the amount of DNA as a consequence of bacterial growth and will decay until zero after a few weeks (Figure 1).

### BRIEF DESCRIPTION OF THE DRAWING

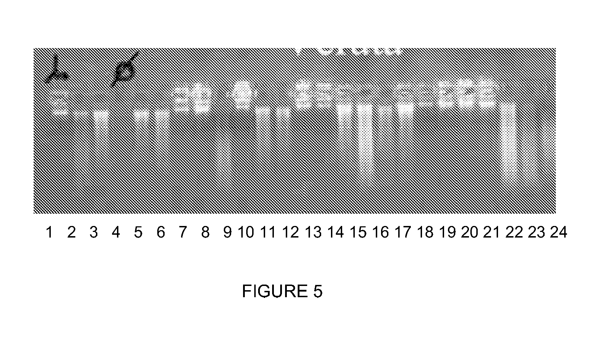

Figure 1 shows the absorbance of the DNA extracted from samples preserved or not in the solution disclosed herein and maintained at +5°C during 8 years.
Figure 2 shows the DNA extracted 48h and 8 months after sampling blood preserved or not in the solution according with the invention which contains EDTA, NaF and Thymol as described herein, or preserved in EDTA K₃ maintained at +5°C or preserved in EDTA K₃ at -20°C.
   1: λ marker
   2-7: blood mixed with the invention
   8-22: blood + EDTA K₃ refrigerated
   23-36: blood + EDTA K₃ frozen
Figure 3: shows the results when samples are extracted (blood with alcaline lysis and tissue with phenol chlorophorm) after maintaining the sample during 8 months at +5°C in the solution described herein and which contains EDTA, NaF and Thymol at pH 8.
   1: λ marker
   2-6: blood
   7-11: muscle
   12-15: hair
   16-20: cartilage
Figure 4 shows the DNA extracted from different samples conserved without any additive after maintenance during 48h (1-7) and without additive and frozen (8-12), visualised in a 0.6% agarose gel
   1: λ marker
   2-3: blood
   4-5: muscle
   6: hair
   7: cartilage
   8-9: blood
   10: muscle
   11: hair
   12: cartilage
Figure 5 shows the DNA extracted from different samples (blood, muscle, cartilage, hair) preserved during 8 months in EDTA K₃ at -20°C.
   1: λ marker
   2-8: blood
   9-14: muscle
   15-21: cartilage
   22-24: hair

### DETAILED DESCRIPTION OF THE INVENTION

As a result of the efficiency of the solution explained herein, different experiments have been performed where different biological tissues, different types of treatments one of which is the solution here disclosed, and different times since sampling has been performed, have been considered as principal effects.

Biological material is blood, ear cartilage, hair and muscle.

For each tissue, 50 animals have been analysed, each of them has 5 replicas.

Sample conservation has been performed at three temperatures (room temperature, refrigerated between 5° and 7°C and frozen at -20°C) and with three different treatments (no additive, with EDTA K₃ and with the solution herein disclosed).

Samples where maintained during a minimum of 48h from sampling until ending the 8 month period.

### Example 1.

A method to extract genomic DNA is presented from blood samples maintained at room temperature and preserved in the solution disclosed herein.

The solution herein described consists in 1 gr EDTA trisodium, 100 mgrs NaF and 0,1 mgr Thymol and pH corrected to 8, is introduced in a 5 ml vacuum tube. Five ml of blood are sampled by venopunction and the tube is thoroughly mixed by inversion. The tube is maintained at room temperature during one week and then at 5°C indefinitely. Tubes are submitted to room temperature 16h before extracting DNA in order to get a good homogenization of blood and solution.

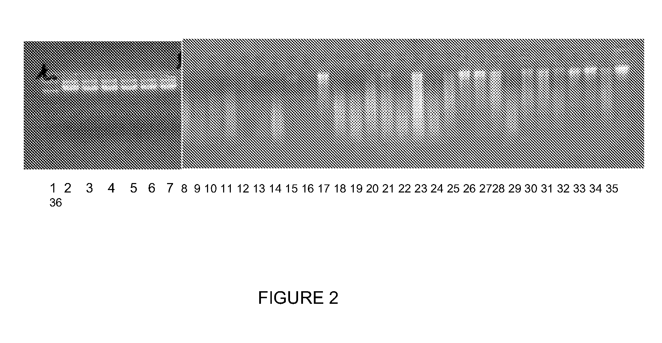

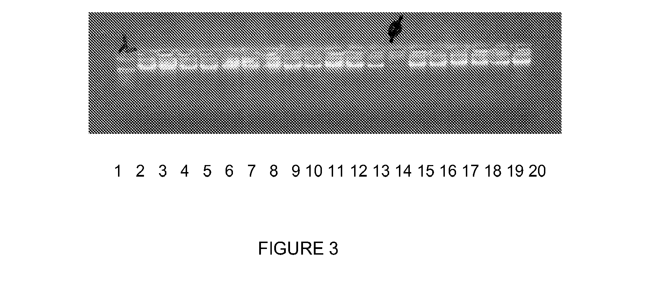

To extract high quantities of DNA (more then 5 µg of DNA), an aliquot of between 1 ml and 3 ml of blood + solution is taken. Same volume of Tris CIH 10 mM + EDTA 10 mM is added, mixed and centrifuged during 3 minutes. Supernatant is discarded without touching pellet. This step is repeated adding this time Tris ClH 10 mM + EDTA 1 mM. After discardig supernatant pellet is incubated in a lysis solution consisting in NaCl 0.1 M and EDTA 25 mM, 12.5 µl SDS 10%, 0.1 mg Proteinase K at 37°C during two or more hours. Then phenol + chlorophorm + isoamilic is added in a proportion of 25:24:1. After centrifugation during 10 minutes, supernatant is moved to a new tube and DNA is precipitated with Ethanol 100% after addition of 40 µl NaAc 3 M pH=5.2 (Figures 2 and 3).

### Example 2:

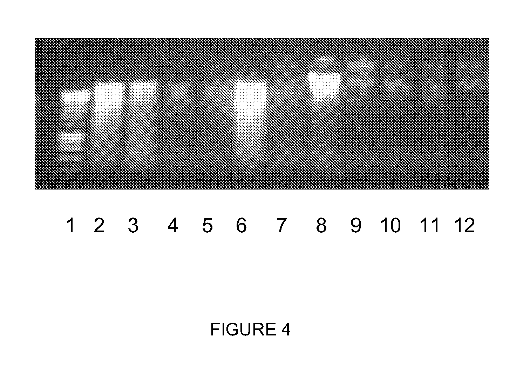

In this example, tissue samples were 500 mgr of muscle, 50 mgr of cartilage. The different tissues were mixed to a solution containing NaCl 0.14 M; MgAc 1.5 mM; ClK 5 mM; SDS 1% and a manual homogenisation treatment during 1 minute. Phenol-chlorophorm-isoamilic is that added in a proportion 25:24:1. After 10 minutes shaking, centrifugation is performed during 5 minutes and supernatant (aqueous phase) is moved to a new tube and DNA is precipitated with Ethanol 100% after addition of 40 µl NaAc 3 M pH=5.2. The result of this extraction can be seen in Figure 4.

### Example 3:

A genomic DNA extraction method is presented from a blood sample contained in the solution herein disclosed.

The solution herein described consists in 1 gr EDTA trisodium, 100 mgrs NaF and 0,1 mgr Thymol and pH corrected to 8, is introduced in a 5 ml vacuum tube.

Five ml of blood are sampled by venopunction and the tube is mixed by inversion. The tube is maintained at ambient temperature indefinitely. On arrival to the extraction laboratory, maintenance at +5°C is recommended. Before extracting DNA, tubes are submitted 16h to room temperature in order to get a good homogenisation of blood and solution. An amount of 50 µl (for amplification of one or two genes), or 100 µl( for an hybridisation test) is taken with a micropipette and passed to a new tube adding 100 µl of distilled H2O. After mixing and 3 minutes centrifugation, supernatant is discarded and this step is repeated twice more after which 100 µl of NaOH 0.25 M is added, incubated for 15 minutes. Then 100 µl of CIH 0.25 M is added and then 100 µl Tris CIH 0.1 M pH = 8.5 then centrifuged and 2 to 5 µl of the supernatant are taken for a PCR amplification. See Figures 2 and 3.

## Claims

1. A solution for the indefinite maintenance of nucleic acids in their cell of origin composed by the following reactive:
• EDTA (Ethylen-diamine-tetraacetic acid) in a quantity which varies from 0.39 M and 0.56 M in its trisodium form (Na₃).
• Sodium fluoride (NaF) at a molarity which varies from 0.33 M to 0.48 M.
• Saturation of this solution with thymol (extracted from *Thymus vulgaris* L.) approximately 0.0001 M.
• Its equilibration with ClH to pH = 8.

2. The solution for the indefinite maintenance of nucleic acids in the cell of origin thereof, according to claim 1, wherein the amount of EDTA in one litter solution varies between 140 gr. and 200 gr.

3. Solution for the indefinite maintenance of nucleic acids in the cell of origin thereof, according to claim 1, wherein the amount of NaF in one litter solution varies from 14 gr. and 20 gr.

4. Solution for the indefinite maintenance of nucleic acids in the cell of origin thereof according to claim 1, wherein the amount of thymol in one litter solution varies from 5 to 10 mg.

5. Solution for the indefinite maintenance of nucleic acids in the cell of origin thereof according to claim 1, wherein pH is equal to 8.

6. Solution for the indefinite maintenance of nucleic acids in the cell of origin thereof, according to the above claims, wherein when it is added to a biological sample obtained from an individual either eukaryote or prokaryote, this is maintained at a temperature equal or superior to +5°C and in such conditions that quality of the nucleic acids extracted later will not be affected, guaranteeing its integrity independently of the time elapsed between sample obtaining and nucleic acids extraction.

7. Solution for the indefinite maintenance of nucleic acids in the cell of origin thereof according to the above claims wherein the biological sample can be blood, an organ or fragment of it, a healthy tissue or not (tumour or inflammatory), skin, feather, bone marrow, egg, blood serum, amniotic liquid, semen, saliva, vaginal fluid, sweat, faeces, urine, hair or a cell culture.

8. Solution for the indefinite maintenance of nucleic acids in the cell of origin thereof, according to the above claims, wherein the solution can be an aqueous, solid or micronised.
